# EUROPÄISCHE PATENTSCHRIFT

(11) **EP 0 413 201 B1**
(45) Veröffentlichungstag und Bekanntmachung des Hinweises auf die Patenterteilung: **26.10.1994**
(21) Anmeldenummer: 90114802.3
(22) Anmeldetag: 02.08.1990
(51) Int. Cl.: C07C 309/26, C07C 303/20, C07C 303/22, G03F 7/022

(54) **Verfahren zur Herstellung von 7-Hydroxy-1,2-naphthochinon-(2)-diazid-4-sulfonsäure oder ihrer Salze und deren Verwendung**
Process for the preparation of 7-hydroxy-1,2-naphthoquinone-(2)-diazide-4-sulfonic acid or salts thereof and their use
Procédé pour la préparation de l'acide 7-hydroxy-1,2-naphthoquinone-(2)-diazide-4-sulfonique ou ses sels et leur application

(30) Priorität: 12.08.1989 DE 3926774
(43) Veröffentlichungstag der Anmeldung: 20.02.1991
(73) Patentinhaber: HOECHST AKTIENGESELLSCHAFT, 65926 Frankfurt am Main (DE)
(72) Erfinder: Scheler, Siegfried, Dr., Dipl.-chem., D-6200 Wiesbaden-Naurod (DE); Buhr, Gerhard, Dr., Dipl.-Chem., D-6240 Königstein (DE); Bergmann, Klaus, D-6500 Mainz-Bretzenheim (DE)

(56) Entgegenhaltungen:
- EP-A- 0 283 898
- DE-A- 3 837 499
- JOURNAL OF THE AMERICAN CHEMICAL SOCIETY, Band 80, 1958, Seiten 2257-2263,Columbus, Ohio, US; M.P. CAVA et al.: "Condensed cyclobutane aromatic systems; V. The synthesis of some alpha-diazo-indanones: Ring contraction in the indane series"

## Beschreibung

Die vorliegende Erfindung betrifft ein Verfahren zur Herstellung von 7-Hydroxy-1,2-naphthochinon-(2)-diazid-4-sulfonsäure oder ihrer Salze und ihre Verwendung.

Ester, Amide und Hydrazide der 1,2-Naphthochinon-(2)-diazidsulfonsäuren werden schon seit vielen Jahren als lichtempfindliche Verbindungen für strahlungsempfindliche Gemische, wie etwa Photoresists für die Herstellung von Halbleiterbauelementen in der Mikroelektronik oder als Beschichtungslösungen für die Herstellung von photomechanisch verarbeitbaren Druckformen oder Farbprüffolien, eingesetzt. Geeignete Verbindungen und Verarbeitungsverfahren sind beschrieben in: J. Kosar, "Light Sensitive Systems", John Wiley & Sons, New York, Kap. 7.4, 1965, US-PS 4 104 070, US-PS 4 576 901 und EP-A 0 212 482.

Gemäß der EP-A 0 258 727 werden ortho-Naphthochinondiazidsulfonsäureester hergestellt durch Umsetzen eines ortho-Naphthochinondiazidsulfonsäurehalogenids mit einer ein- oder mehrwertigen phenolischen Verbindung in Gegenwart von Ammoniak, Ammoniumsalzen schwacher Säuren oder von aliphatischen Abkömmlingen des Ammoniaks mit 1 bis 3 Kohlenstoffatomen bei einem pH-Wert im Bereich zwischen etwa 1,5 und 8,5. Die Ester lassen sich in einem lichtempfindlichen Gemisch verwenden.

Die Herstellung der diesen bekannten Derivaten zugrunde liegenden 1,2-Naphthochinon-(2)-diazidsulfonsäuren geht aus von der 1-Naphthol-4- bzw. -5-sulfonsäure, die mit Alkalinitrit in verdünnter Mineralsäure nitrosiert wird. Die entstandenen 2-Nitroso-1-naphtholsulfonsäuren werden isoliert, und die nicht umgesetzten Ausgangsstoffe sowie die bei der Nitrosierung entstandenen Nebenprodukte durch Auswaschen oder Umlösen entfernt. Die Nitrosoverbindung wird anschließend in wäßriger Lösung zur entsprechenden Aminoverbindung reduziert. Diese wird isoliert und erneut von nicht umgesetzten Ausgangsstoffen und entstandenen Nebenprodukten durch Digerieren in Wasser oder durch Umlösen befreit. Anschließend wird die Aminoverbindung in Wasser suspendiert und bei einem pH-Wert von 4 - 6 mit Alkalinitrit in Anwesenheit von Cu (II)-Salzen diazotiert. In den meisten Fällen müssen die auf diese Weise gewonnenen 1,2-Naphthochinon-(2)-diazidsulfonsäuren noch durch Umlösen oder Umkristallisieren von den bei der Diazotierung entstandenen dunkel gefärbten Nebenprodukten befreit werden.

Der Nachteil dieses Herstellungsverfahrens besteht im wesentlichen darin, daß die bei den einzelnen Reaktionsstufen nicht umgesetzten Einsatzstoffe und die entstandenen Nebenprodukte vom gewünschten Hauptprodukt durch einen zusätzlichen Reinigungsschritt abgetrennt werden müssen. Damit verbunden sind niedrige Ausbeuten, eine nicht immer befriedigende Produktqualität und hohe Herstellungskosten.

Aus der EP-A 0 283 898 ist ein Verfahren zur Herstellung von gegebenenfalls durch Halogen, Nitro- oder Alkylgruppen substituierten Benzo- und Naphthochinon-diazid-sulfonsäuren und deren Salzen bekannt. Man geht hierbei aus von einer Arylsulfonsäure mit mindestens einer Hydroxygruppe, nitrosiert in bekannter Weise, reduziert die entstandene Nitrosoverbindung im alkalischen pH-Bereich und wandelt anschließend die Aminoverbindung in ein Sulfamatderivat um, das anschließend mit einem Diazotierungsagenz gemischt wird. Nach dem Ansäuern der Mischung erhält man die Benzo- bzw. Naphthochinon-diazidsulfonsäuren. Die nach jedem Reaktionsschritt entstandenen Reaktionsprodukte werden nicht zwischenisoliert, sondern verbleiben zur Weiterreaktion in Lösung ("Eintopfreaktion"). Nebenprodukte und Verunreinigungen, die bei den einzelnen Verfahrensschritten anfallen, können durch Filtration der Reaktionslösung in befriedigender Weise abgetrennt werden.

Nachteilig an diesem Einstufenverfahren ist, daß die verschiedenen Verfahrensschritte nur in einem relativ kleinen pH-Bereich durchführbar sind und die Reaktionszeiten und Reaktionstemperaturen sehr exakt eingehalten werden müssen. Die nach diesem Verfahren erhaltenen Endprodukte sind in der Regel nicht frei von isomeren Verbindungen. Deshalb ist ein universeller technischer Einsatz der nach diesem Verfahren hergestellten Verbindungen eingeschränkt.

In der nicht vorveröffentlichten deutschen Patentanmeldung, Aktenzeichen P 38 37 499.4 wird ein Verfahren zur Herstellung von Estern kernsubstituierter 1,2-Naphthochinon-(2)-diazid-4-sulfonsäuren beschrieben, bei dem als Zwischenstufen 1,2-Naphthochinon-(2)-diazid-4-sulfonsäuren anfallen, die in 5-, 6-, 7- oder 8-Position durch Halogen, Alkoxy oder Alkoxycarbonyl substituiert sind.

Man geht hierbei aus von einem entsprechend substituierten 2-Naphthol, nitrosiert in 1-Position, sulfoniert mit Alkalihydrogensulfit in 4-Position und reduziert anschließend die Nitrosogruppe durch Ansäuern mit Mineralsäure bei einem pH-Wert ≦ 7 zur Aminogruppe, oxidiert die 2-Amino-1-naphthol-4-sulfonsäure zur entsprechenden 1,2-Naphthochinon-4-sulfonsäure und setzt diese mit p-Toluolsulfonsäurehydrazid in einem organischen Lösungsmittel bei Temperaturen von 20 - 100 °C zu der entsprechenden kernsubstituierten 1,2-Naphthochinon-(2)-diazid-4-sulfonsäure um.

Durch Chlorierung mit Chlorsulfonsäure bzw. einem Gemisch aus Chlorsulfonsäure/Thionylchlorid erhält man in bekannter Weise das Sulfonsäurechlorid, das anschließend mit einer phenolischen Komponente zu den entsprechenden kernsubstituierten 1,2-Naphthochinon-(2)-diazid-4-sulfonsäureestern kondensiert wird. Die einzelnen Verfahrensschritte zur Herstellung der Zwischen- und Endprodukte sind aus der Literatur bekannt. Das Verfahren ist abhängig von der relativ schweren Zugänglichkeit der als Ausgangsstoffe verwendeten substituierten 2-Naphthole. Beispielsweise erhält man 7-Alkoxy-2-naphthol nach bekannten Verfahren durch Monoalkylierung von 2,7-Dihydroxynaphthalin in einer Ausbeute von nur etwa 50-55 % d. Theorie. Die Ausbeuten der anschließenden Reaktionsstufen - Nitrosierung, Sulfonierung und Reduktion, Oxidation, Einführung der Diazogruppe - sind zufriedenstellend. Trotz Verzicht auf zusätzliche Reinigung der Zwischenstufen ist die Gesamtausbeute an 7-Alkoxy-1,2-napthochinon-(2)-diazid-4-sulfonsäure, bezogen auf das eingesetzte 2,7-Dihydroxynaphthalin, noch nicht zufriedenstellend, so daß die Produktionskosten für die herstellbaren Sulfonsäureester relativ hoch sind.

Ein weiteres Verfahren zur Herstellung kernsubstituierter 1,2-Naphthochinon-(2)-diazid-4-sulfonsäuren, die zur Synthese der entsprechenden Ester und Amide verwendet werden können, wird in der nicht vorveröffentlichten deutschen Patentanmeldung, Aktenzeichen P 38 37 500.1, angegeben. Zur Herstellung von 7-Methoxy-1,2-naphthochinon-(2)-diazid-4-sulfonsäure, geht man beispielsweise von käuflichem 1-Acetylamino-7-naphthol aus. Dieses kann in einer 7-stufigen Reaktionsfolge - Methylierung der phenolischen Hydroxylgruppe, Abspaltung der Acetylgruppe und Herstellung von 1-Amino-7-methoxynaphthalin-Hydrogensulfat, Sulfonierung in 4-Position durch trockenes Erhitzen ("Backreaktion"), Austausch der Aminogruppe durch eine Hydroxylgruppe ("Bucherer Reaktion"), Nitrosierung in 2-Position, Reduktion der Nitroso- zur Aminogruppe, Diazotierung - zu der gewünschten Verbindung umgesetzt werden.

Wegen der Mehrstufigkeit und wegen betriebstechnisch schwierig durchführbarer Verfahrensschritte sowie der nicht immer zufriedenstellenden Ausbeute einzelner Zwischenstufen ist auch dieses Herstellungsverfahren technisch problematisch.

Für die Herstellung von Photolacken ist es jedoch von größter Wichtigkeit, über ein wirtschaftliches Syntheseverfahren der strahlungsempfindlichen Komponenten zu verfügen.

Aus DD-A 263 982 ist die Herstellung von 2-Diazo-1-oxo-1,2-dihydronaphthalinderivaten, ausgehend von 1,7-Dihydroxynaphthalinderivaten bekannt. Die hierzu als Ausgangsmaterial verwendete 1,7-Dihydroxynaphthalin-4-sulfonsäure ist jedoch schwer zugänglich, so daß die Durchführung des Gesamtverfahrens extrem aufwendig ist. Außerdem ist innerhalb des Verfahrens die Diazotierung der Aminozwischenstufe nur unter definierten P_{H}- und Temperaturbedingungen und in Anwesenheit von Schwermetallsalzen in akzeptabler Qualität und in nur geringer Ausbeute möglich.

Die Ester und Amide der 7-Methoxy-1,2-naphthochinon-(2)-diazid-4-sulfonsäure sind wegen der längerwelligen Verschiebung ihrer Absorption und ihres hohen Umkehrpotentials hervorragend für die Verwendung in Photoresistschichten geeignet, die im g-line (436 nm)- und i-line (365 nm)-Bereich strukturiert und sowohl positiv als auch negativ verarbeitet werden können.

Aufgabe der Erfindung war es deshalb, ein Verfahren zur Herstellung von in 7-Position substituierter 1,2-Naphthochinon-(2)-diazid-4-sulfonsäure und deren Derivaten anzugeben, welches die Nachteile der bekannten Verfahren vermeidet, und wodurch Verbindungen wie Säuren, Salze und die für Photolacke bevorzugten Ester und Amide nach produktionstechnisch einfachen Verfahrensschritten preisgünstig und in guter Ausbeute hergestellt werden können.

Die Erfindung betrifft ein Verfahren zur Herstellung von Verbindungen der allgemeinen Formel Z 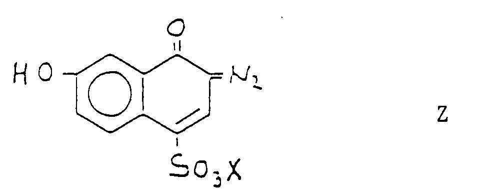
in der
- X =: Wasserstoff, Metall, vorzugsweise Alkali- oder Erdalkalimetall, insbesondere Natrium oder Kalium oder eine Ammoniumgruppe bedeutet,
bei dem man
1) 2,7-Dihydroxynaphthalin nitrosiert,
2) das entstandene 2,7-Dihydroxy-1-nitrosonaphthalin (I) mit Alkalihydrogensulfit sulfoniert und die gebildete Bisulfit Additionsverbindung in saurer Lösung zur 2,7-Dihydroxy-1-aminonaphthalin-4-sulfonsäure (II) reduziert,
3) diese zur 7-Hydroxy-1,2-naphthochinon-4-sulfonsäure (III) oxidiert und als Salz abscheidet und
4) dieses Salz mit einem Arylsulfonsäurehydrazid zu dem entsprechenden Salz der 7-Hydroxy-1,2-naphthochinon-(2)-diazid-4-sulfonsäure (IV) umsetzt und dieses isoliert.

Die Verbindungen sind dem Reaktionsschema zu entnehmen. 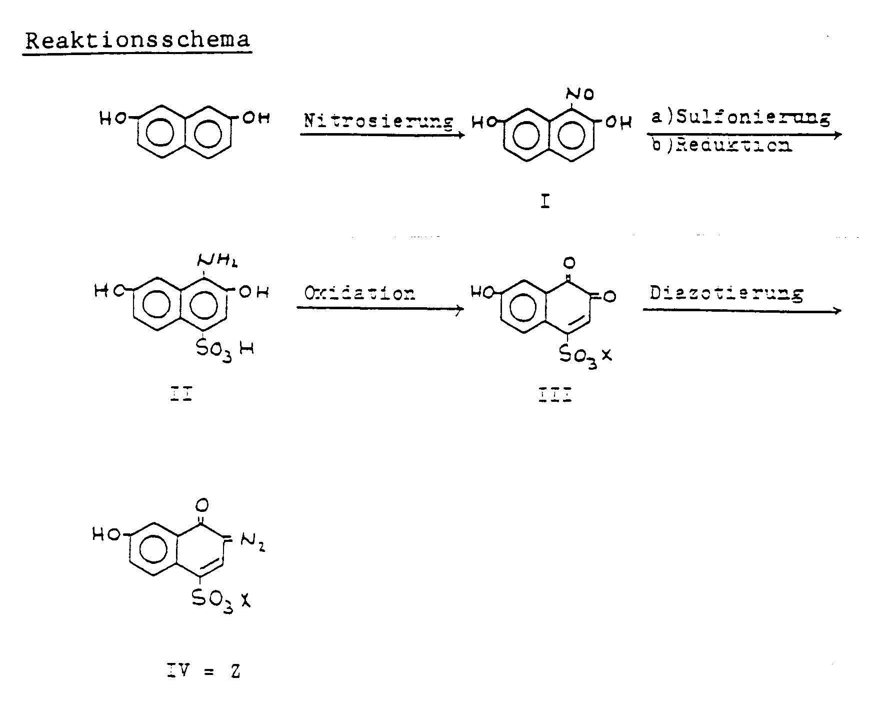
Vorteilhaft an dem erfindungsgemäßen Verfahren ist, daß zusätzliche Reinigungsschritte für die anfallenden Zwischenprodukte entfallen.

Die Nitrosierung erfolgt vorzugsweise in essigsaurer wäßriger Suspension mit Alkalinitrit bei Temperaturen von + 5 bis - 10 °C. Die Nitrosoverbindung wird anschließend isoliert. Die Sulfonierung mit Alkalihydrogensulfit oder Alkalidisulfit gelingt in wäßriger Phase in einem pH-Bereich von 5 - 7. Die entstandenen Bisulfit-Additionsverbindung wird ohne Zwischenisolierung in mineralsaurer wäßiger Lösung bei Temperaturen zwischen 20 und 60 °C zu der entsprechenden Aminoverbindung reduziert. Die Oxidation wird vorzugsweise mit wäßriger Salpetersäure von 15 - 25 Gewichtsprozent, bei Temperaturen zwischen 15 und 25 °C durchgeführt. Die entstandene Naphthochinonsulfonsäure wird vorzugsweise als Ammonium-, insbesondere Kaliumsalz abgeschieden und isoliert. Die Umsetzung zum Diazid erfolgt mit p-Toluolsulfonsäurehydrazid in wäßriger oder organischer Phase, vorzugsweise in Methanol, bei Temperaturen zwischen 20 und 70 °C, vorzugsweise zwischen 25 und 30 °C zu dem entsprechenden Salz der 7-Hydroxy-1,2-naphthochinon-(2)-diazid-4-sulfonsäure, das anschließend isoliert wird.

Die erfindungsgemäßen Verbindungen können als solche, bevorzugt jedoch als Zwischenprodukte bzw. Ausgangsstoffe über die Zwischenstufe der Sulfonsäurechloride für die Her-stellung technisch interessanter lichtempfindlicher Ver bindungen und strahlungsempfindlicher Gemische und Materialien verwendet werden.

Das erfindungsgemäße Herstellungsverfahren geht aus von handelsüblichem 2,7-Dihydroxynaphthalin, das in bekannter Weise durch Nitrosierung mit Natriumnitrit bei Temperaturen zwischen 0 und 5 °C entweder in mineralsaurer wäßriger Suspension (Clausius, B. 23, 517 (1890); Leonhardt & Co D.R.P. 55 204 (1889)) oder in essigsaurer Lösung (Kaufler u. Bräuer, B. 40, 3275 (1907)) zu 2,7-Dihydroxy-1-nitrosonaphthalin (I), bzw. dem tautomeren 7-Hydroxy-1,2-naphthochinon-(1)-oxim (I a), umgesetzt werden kann. Nach beiden bekannten Verfahren sind Ausheute und Qualität des 2,7-Dihydroxy-1-nitrosonaphthalins (I) nicht zufriedenstellend.

Völlig überraschend und in nahezu quantitativer Umsetzung und sehr guter Qualität verläuft jedoch die Nitrosierung von 2,7-Dihydroxynaphthalin analog der von Gates und Webb in J. Am. Chem. Soc. 80, 1186 (1958) für die Herstellung von 6-Methoxy-1-nitroso-2-naphthol beschriebenen Methode. Hiernach wird eine feindisperse Suspension von 2,7-Dihydroxynaphthalin, die man durch Ausfällen von 2,7-Dihydroxynaphthalin mit Eis aus einer warmen essigsauren Lösung erhält, unter kräftigem Rühren bei + 5 bis -10 °C mit Natriumnitrit versetzt. Die entstandene dunkelrote mikrokristalline Nitroso-/Oximverbindung (I/Ia) wird abgesaugt, mit Wasser neutral gewaschen und gegebenenfalls getrocknet. Das nach dieser Verfahrensweise erhaltene Reaktionsprodukt wird ohne Reinigung, vorzugsweise als wasserfeuchtes Produkt weiterverarbeitet. 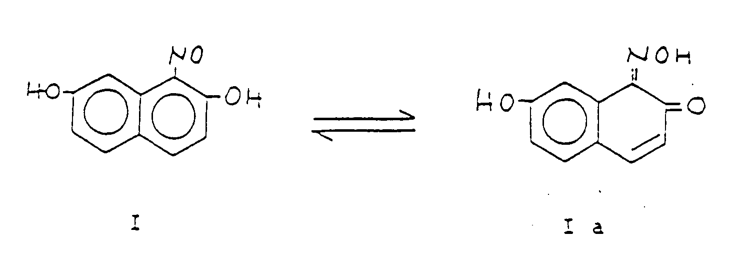
In der zweiten Reaktionsstufe wird das in der Regel noch wasserfeuchte 2,7-Dihydroxy-1-nitrosonaphthalin (I), das in der tautomeren Oximform (Ia) reagiert, nach Böninger, Ber. 27,3050 (1894) zunächst in handelsüblicher 37 %iger wäßriger Natriumhydrogensulfitlösung suspendiert und bei 20 - 25 °C bis zur vollständigen Lösung gerührt. Die hierbei entstandene Bisulfit-Additionsverbindung (IIa) wird nicht isoliert, sondern die braune Reaktionslösung mit Salzsäure angesäuert und auf ca. 25 - 60 °C erwärmt, wobei durch Reduktion der Nitrosogruppe und Aromatisierung des cycloaliphatischen Ringsystems die 2,7-Dihydroxy-1-aminonaphthalin-4-sulfonsäure (II) gebildet wird. Man isoliert die Aminonaphtholsulfonsäure (II), indem man die entstandenen hellgrauen Kristalle absaugt, diese auf der Filternutsche zuerst mit Wasser und dann mit Methanol wäscht und gegebenenfalls trocknet. Das so hergestellte Reaktionsprodukt wird ohne weitere Reinigung, gegebenenfalls noch feucht, für die nächste Reaktionsstufe eingesetzt. Die Ausbeute an Reaktionsprodukt II liegt bei 90 - 95 % d. Theorie. 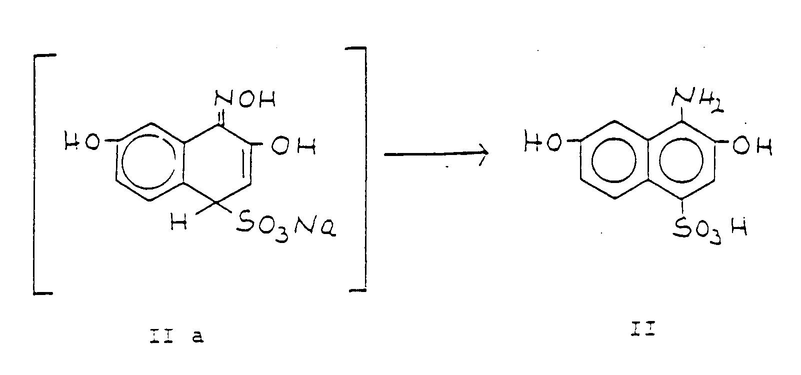
Vorteilhafter bei der praktischen Durchführung der Reaktion ist die Verwendung von festem, lagerstabilem Natriumdisulfit anstelle der käuflichen Natriumhydrogensulfit-Lösung.

In der dritten Reaktionsstufe wird die 2,7-Dihydroxy-1-aminonaphthalin-4-sulfonsäure (II) mit einem Oxidationsmittel zu 7-Hydroxy-1,2-naphthochinon-4-sulfonsäure (III) oxidiert.

Geeignete Oxidationsmittel sind z.B. Kaliumperoxidisulfat (K₂S₂O₈), Cr(VI)-oxid, Pb(IV)-oxid, Fe(III)-chlorid, Chlor, Brom oder salpetrige Säure. Vorzugsweise wird jedoch verdünnte Salpetersäure als Oxidationmittel verwendet. Die Isolierung der 7-Hydroxy-1,2-naphthochinon-4-sulfonsäure (III) erfolgt üblicherweise als Ammonium-oder als Alkalisalz.

Bei Verwendung von Salpetersäure verfährt man zweckmäßigerweise analog der von Martin und Fieser in Org. Synth. Coll. Vol. III, 633 (1955) beschriebenen Verfahrensweise. Die Oxidation erfolgt vorzugsweise bei Temperaturen zwischen 15 und 25 °C, wobei man in der Regel die feste Aminonaphtholsulfonsäure unter Rühren portionsweise in die Salpetersäure einträgt. Man arbeitet zweckmäßigerweise mit verdünnter Salpetersäure, um das als Suspension vorliegende Reaktionsgemisch rührfähig zu halten und die exotherm ablaufende Oxidation durch Außenkühlung besser auf Raumtemperatur begrenzen zu können. Besonders bewährt hat sich eine 15 bis 25 vorzugsweise 18 - 22 %ige Salpetersäure als Oxidationsmittel. Die entstandene 7-Hydroxy-1,2-naphthochinon-4-sulfonsäure (III) wird vorzugsweise als Ammonium- oder Kaliumsalz durch Aussalzen mit Ammonium-bzw. Kaliumchlorid gefällt und durch Filtration isoliert. Der Filterrückstand wird mit einer gesättigten Ammonium- bzw. Kaliumchloridlösung und anschließend mit Ethanol gewaschen und gegebenenfalls bei 20 - 40 °C getrocknet. Die auf diese Weise hergestellten Salze der 7-Hydroxy-1,2-naphthochinon-4-sulfonsäure enthalten in der Regel geringe Anteile (ca. 2 - 3 %) an 7-Hydroxy-1,2-naphthochinon-(1)-diazid-4-sulfonsäure (IV a). Diese entsteht in einer Nebenreaktion durch Diazotierung der eingesetzten 2,7-Dihydroxy-1-aminonaphthalin-4-sulfonsäure mit salpetriger Säure, die bei Anwendung von Salpetersäure im Oxidationsmedium vorhanden ist. Dieses nur in geringer Menge gebildete Nebenprodukt stört jedoch die nachfolgende Umsetzung zur 7-Hydroxy-1,2-naphthochinon-(2)-diazid-4 sulfonsäure (IV) nicht, da die Salze der isomeren 7-Hydroxy-1,2-naphthochinon-(1)-diazid-4-sulfonsäure (IV a) besser löslich sind als die gewünschten Salze der 7-Hydroxy-1,2-naphthochinon-(2)-diazid-4-sulfonsäure (IV). Bei Anwendung von höher konzentrierter Salpetersäure steigt der Anteil an 7-Hydroxy-1,2-naphthochinon-(1)-diazid-4-sulfonsäure (IV a).

Deutlich größere Mengen (ca. 20 - 40 %) an 7-Hydroxy-1,2-naphthochinon-(1)-diazid-4-sulfonsäure (IV a) entstehen nach der von Böninger in Ber. 27, 3050, (1894) beschriebenen Oxiationsmethode in verdünnter Salzsäure mit Natriumnitrit bei 0 bis 5 °C. Aus der rotbraunen Lösung werden nach dieser bekannten Verfahrensweise durch Aussalzen mit Kaliumchlorid die Kaliumsalze der gewünschten 7-Hydroxy-1,2-naphthochinon-4-sulfonsäure (III) und der unerwünschten 7-Hydroxy-1,2naphthochinon-(1)-diazid-4-sulfonsäure (IV a) zusammen ausgefällt. Eine Trennung der Kaliumsalze dieser beiden Sulfonsäuren durch fraktionierte Kristallisation ist grundsätzlich möglich, jedoch sehr verlustreich. Für ein technisches Produktionsverfahren ist diese Oxidationsmethode deshalb ungeeignet.

In der vierten Reaktionsstufe wird das Ammonium- oder das Alkalisalz, vorzugsweise das Kaliumsalz der 7-Hydroxy-1,2-naphthochinon-4-sulfonsäure (III), mit einem Arylsulfonsäurehydrazid, z.B. p-Toluolsulfonsäurehydrazid, in wäßriger, vorzugsweise jedoch in alkoholischer Suspension, z.B. Methanol, bei Temperaturen zwischen 20 und 70 °C, vorzugsweise 20 - 30 °C, mit hoher Regioselektivität zu den entsprechenden Salzen der 7-Hydroxy-1,2-naphthochinon-(2)-diazid-4-sulfonsäure (IV) umgesetzt. Die noch zu erwartenden isomeren Salze der 7-Hydroxy-1,2-naphthochinon-(1)-diazid-4-sulfonsäure (IV a) entstehen unter diesen Reaktionsbedingungen nur in äußerst geringen Anteilen (ca. 1 - 2 %). 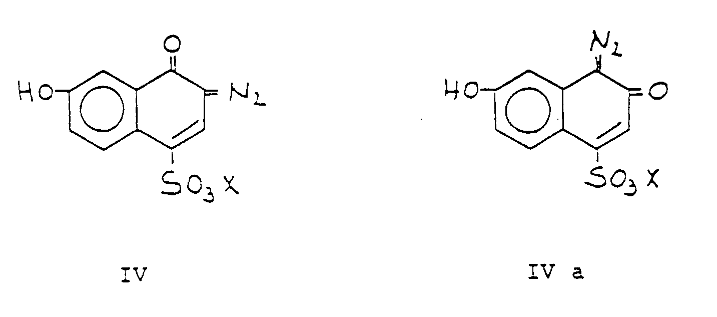
Die ausgezeichnete Regioselektivität bei dieser Umsetzung ist insofern überraschend, da sich das Isomerenverhältnis der von Horner und Dürckheimer hergestellten und in Chem. Ber. 95, 1206 (1962) beschriebenen substituierten o-Benzochinondiazide von dem Isomerenverhältnis der o.g. substituierten o-Naphthochinondiaziden z.T. sehr deutlich unterscheidet.

Die 7-Hydroxy-1,2-naphthochinon-(2)-diazid-4-sulfonsäure (IV) und ihre Salze sind in der Literatur bisher noch nicht beschrieben. Durch Reaktionen an der phenolischen Hydroxylgruppe, z.B. Alkylierung oder Acylierung, oder an der Sulfonsäuregruppe, z.B. Chlorierung, gelingt es jetzt auf sehr einfache Weise, die substituierten 1,2-Naphthochinon-(2)-diazid-4-sulfonsäuren bzw. 7-Hydroxy-1,2-naphthochinon-(2)-diazid-4-sulfonsäurechlorid herzustellen.

Der Austausch eines Sauerstoffatoms bei α-Diketonen durch die Diazogruppe mit Arylsulfonsäurehydrazid ist aus der Literatur als "Bamford-Stevens-Reaktion" bekannt. Bei dieser Umsetzung entsteht intermediär ein Diketonarylhydrazon, das mit oder ohne Verwendung von Alkali unter sehr einfachen und milden Reaktionsbedingungen zu einer α-Diazocarbonylverbindung gespalten werden kann.

In der DE-B 11 26 541 wird diese Reaktion für die Herstellung von 6-Nitro-1,2-naphthochinon-(2)-diazid-4,8-disulfonsäure aus 6-Nitro-1,2-naphthochinon-4,8-disulfonsäure durch Umsetzung mit Arylsulfonsäurehydrazid und anschließende Spaltung des primär gebildeten Hydrazons in wäßrig alkalischem Medium beschrieben. Eine Isolierung des Naphthochinondiazid-Derivats in Substanz ist für das in dieser Anmeldung beschriebene Herstellungsverfahren für spezielle Azofarbstoffe nicht erforderlich.

Eine weitere Anwendung der "Bamford-Stevens-Reaktion" wird von Süs, Steppan und Dietrich in Liebigs Ann. Chem. 617, 20 (1958) für die Herstellung polycyclischer aromatischer o-Chinondiazide, z.B. Phenanthrenchinon-(9,10)-und Chrysenchinon-(5,6)-diazid, beschrieben. Man erhält diese o-Chinondiazide durch Umsetzung der entsprechenden polycyclischen o-Chinone mit p-Toluolsulfonsäurehydrazid in Äthanol bei Temperaturen zwischen 45 und 60 °C und anschließende Spaltung der intermediär gebildeten Toluolsulfonsäurehydrazone ohne Anwendung von Alkali.

Über die Anwendungsbreite und über die verschiedenen Verfahrensvarianten der "Bamford-Stevens-Reaktion" wird in der Fachliteratur zusammenfassend berichtet (M. Regitz, "Diazoalkane", Kap. 5.3, 129 (1977) oder Houben-Weyl, "Aromatische Diazoniumsalze", Bd. 10/3, 84).

Bei der Herstellung des Ammonium- bzw. Kaliumsalzes der 7-Hydroxy-1,2-naphthochinon-(2)-diazid-4-sulfonsäure (IV) geht man zweckmäßigerweise so vor, daß man die entsprechenden Salze der 7-Hydroxy-1,2-naphthochinon-4-sulfonsäure (III) in Wasser oder einem polaren organischen Lösemittel) vorzugsweise Methanol, suspendiert und bei Temperaturen zwischen 15 und 30 °C das p-Toluolsulfonsäurehydrazid unter Rühren zudosiert. Das Reaktionsgemisch wird unter fortgesetztem Rühren auf 20 bis 40 °C erwärmt. Wird ein polares organisches Lösemittel, z.B. Methanol, als Reaktionsmedium verwendet, wandelt sich die anfangs dunkelrote Suspension der Salze der 7-Hydroxy-1,2-naphthochinon-4-sulfonsäure in der Regel ohne erkennbare Lösung in eine gelbe Suspension um. Die nach dieser Verfahrensweise in hohen Ausbeuten anfallenden Ammonium- bzw. Kaliumsalze der 7-Hydroxy-1,2-naphthochinon-(2)-diazid-4-sulfonsäure werden aus dem Reaktionsgemisch durch einfache Filtration isoliert und mit reichlich Methanol gewaschen. Eine zusätzliche Reinigung, z.B. Umfällen oder Umkristallisieren, ist nicht erforderlich.

Die Verwendung eines polaren Lösemittels als Reaktionsmedium, wie z.B. Methanol, ist deshalb besonders vorteilhaft, weil das gebildete Ammonium- oder Kaliumsalz der 7-Hydroxy-1,2-naphthochinon-(2)-diazid-4-sulfonsäure in diesem Lösemittel nur sehr schwer löslich ist. Die geringen Anteile an Verunreinigungen, die vom Einsatzmaterial herrühren, und die bei der Hydrazonspaltung entstehende p-Toluolsulfinsäure sind jedoch in Methanol sehr gut löslich, so daß die Ausbeute an reinem Reaktionsprodukt nach dieser Verfahrensweise sehr hoch ist.

Bei der wäßrigen Verfahrensweise wird die nach der Umsetzung mit p-Toluolsulfonsäurehydrazid entstandene gelbe Suspension durch Erwärmen auf Temperaturen zwischen 40 und 50 °C gelöst, und die gelbbraune Lösung mit einem Adsorptionsmittel, z.B. Aktivkohle, von wenig dunklen Produkten befreit und aus dem klaren Filtrat durch Aussalzen mit Ammonium- oder Kaliumchlorid und Abkühlen auf 0 - 5 °C das Reaktionsprodukt ausgefällt. Die Ausbeute und Reinheit ist nach dieser Verfahrensweise geringer als nach der bevorzugten Methode in Methanol als Reaktionsmedium.

Die erfindungsgemäßen Verbindungen der allgemeinen Formel Z können in lichtempfindlichen Gemischen oder als Zwischenstufen für die Herstellung von Estern und Amiden der in 7-Position substituierten 1,2-Naphthochinon-(2)-diazid-4-sulfonsäuren verwendet werden. Die Ester und Amide dieser substituierten Naphthochinondiazide können als lichtempfindliche Verbindungen für strahlungsempfindliche Gemische, wie etwa Photoresists für die Herstellung von Halbleiterbauelenenten in der Mikroelektronik oder für Beschichtungslösungen zur Herstellung von photomechanisch verarbeitbaren Druckformen oder Farbprüffolien, eingesetzt werden.

Die Erfindung wird durch die folgenden Beispiele näher erläutert, ohne sie hierauf zu beschränken.

### Experimenteller Teil

### 2,7-Dihydroxy-1-nitrosonaphthalin (I)

250 g (1,56 Mol) 2,7-Dihydroxynaphthalin (BAYER) wurden in 875 ml Eisessig bei 0 - 90 °C gelöst und durch Zugabe von 2,5 kg gestoßenem Eis wieder ausgefällt, wobei eine sehr feinteilige, hellbeige, viskose Suspension entstand und die Temperatur auf -10 bis -12 °C absank. Unter gutem Rühren und Außenkühlung wurden in diese Suspension 107,88 g (1,56 Mol) festes Natriumnitrit portionsweise eingetragen, eine Stunde nachgerührt und dann weitere 10,7 g (0,56 Mol) festes Natriumnitrit zudosiert. Anschließend wurde noch zwei Stunden bei -8 bis -5 °C nachgerührt, das in dunkelroten Kristallen angefallene 2,7-Dihydroxy-1-nitrosonaphthalin abgesaugt und auf der Nutsche gut abgepreßt. Das noch nutschenfeuchte Reaktionsprodukt wurde dann in 5 l Wasser von 20 - 25 °C ca. eine Stunde gerührt, erneut abgesaugt, gut abgepreßt und 24 Stunden im Umlufttrockenschrank bei 20 - 25 °C getrocknet.
- Ausbeute:: 294 g Reinprodukt (= 99,6 % d. Theorie)

### Kenndaten:

- Aspekt:: dunkelrotes feinteiliges Pulver
- MP:: 140 - 145 °C (Aufhellung)
≧ 195 °C (Verkohlung ohne zu schmelzen)

### Elementaranalyse

| Bruttoformel: C₁₀H₇O₃N | | | |
|---|---|---|---|
| MG: | 189 | | |

| | C | H | N |
|---|---|---|---|
| Ber.: | 63,5 | 3,7 | 7,4 |
| Gef.: | 63,7 | 3,7 | 7,1 |

### 2,7-Dihydroxy-1-aminonaphthalin-4-sulfonsäure x 2H₂O (II)

200 g (1,06 Mol) 2,7-Dihydroxy-1-nitrosonaphthalin (I) wurden in 1,75 l Wasser von 20 - 25 °C suspendiert und unter Rühren 67 g (0.8 Mol) Natriumhydrogencarbonat portionsweise langsam zugegeben, wobei anfängliches Schäumen durch Zugabe von 1 ml n-Octanol verhindert wurde. Anschließend wurden 225 g (1,2 Mol) Natriumdisulfit (Na₂S₂O₅) zugesetzt und 17 Stunden bei 20 - 25 °C gerührt, wobei sich die anfangs rote Suspension in eine braune Lösung umwandelte. Anschließend wurde durch Filtration der Lösung über Aktivkohle von wenig dunklen schmierigen Produkten abgetrennt, das jetzt klare braune Filtrat mit 660 ml 37 %iger Salzsäure bis zur kongosauren Reaktion versetzt und unter Rühren 75 Minuten auf 40 - 50 °C erwärmt. Unter SO₂-Entwicklung fiel bereits während dieser Zeit ein Teil des Reaktionsproduktes aus. Nach Abkühlen des Reaktionsgemisches auf 20 - 25 °C und 24-stündigem Stehen wurden die ausgefallenen hellgrauen Kristalle abgesaugt, gut abgepreßt und auf der Filternutsche zuerst mit 500 ml Wasser und dann mit 1 l Methanol gewaschen bis die ablaufende Methanolphase nur noch schwach gelb gefärbt war. Nach der Trocknung im Umlufttrockenschrank bei 20 - 25 °C wurde die 2,7-Dihydroxy-1-aminonaphthalin-4-sulfonsäure, die noch zwei Mol Kristallwasser enthielt, als hellgraues Pulver erhalten.
- Ausbeute:: 288 g Reinprodukt (= 90,5 % d. Theorie)

### Kenndaten:

- Aspekt:: hellgraues Pulver
- MP:: ≧ 275 °C (Z)

### Elementaranalyse

| Bruttoformel: C₁₀H₉O₅NS x 2H₂O | | | | | |
|---|---|---|---|---|---|
| MG: | 291 | | | | |

| | C | H | N | S | H₂O |
|---|---|---|---|---|---|
| Ber.: | 41,2 | 4,5 | 4,8 | 11,0 | 12,4 |
| Gef.: | 41,8 | 4,2 | 4,6 | 10,9 | 12,1 |

### 7-Hydroxy-1,2-naphthochinon-4-sulfonsäure (K-Salz) x 1 H₂O (III)

219 g (0,662 Mol) 19 %ige Salpetersäure wurden auf 15 °C abgekühlt und 1,5 g (5,2 x 10⁻³ Mol) 2,7-Dihydroxy-1-aminonaphthalin- 4-sulfonsäure x 2H₂O (II) unter Rühren eingetragen. Nach Starten der Oxidationsreaktion mit ca. 1 ml 65 %iger Salpetersäure wurden unter fortgesetztem Rühren jetzt in die entstandene dunkelrote Lösung die restlichen 148,5 g (0,51 Mol) 2,7-Dihydroxy-1-aminonaphthalin-4-sulfonsäure x 2H₂O bei 15 - 20 °C innerhalb von zwei Stunden portionsweise langsam eingetragen. Anfängliches Schäumen konnte durch Zugabe einiger Tropfen n-Octanol stark reduziert werden. War die gesamte 2,7-Dihydroxy-1-aminonaphthalin-4-sulfonsäure x 2H₂O eindosiert, wurde noch 30 Minuten bei 15 - 20 °C nachgerührt und dann die dunkelrote Suspension in 1,6 l Wasser von 50 °C eingerührt. Es entstand eine klare dunkelrote Lösung.

In diese Lösung wurden 170 g Kaliumchlorid in Anteilen von je ca. 25 g eingetragen. Bereits bei Zugabe der ersten 25 g Kaliumchlorid begann die Abscheidung roter glänzender Kristalle aus der Lösung. Das Reaktionsgemisch wurde auf 0 bis 5 °C abgekühlt, nach zwei Stunden abgesaugt und der Nutscheninhalt zuerst mit 80 ml gesättigte Kaliumchloridlösung, dann mit 160 ml Ethanol gewaschen und gut abgepreßt. Anschließend wurde der Filterrückstand im Umlufttrockenschrank bei 20 - 25 °C getrocknet.

Das so hergestellte rohe 7-Hydroxy-1,2-naphthochinon-4-sulfonsäure-(K-Salz) enthielt neben Kaliumchlorid noch geringe Anteile an 7-Hydroxy-1,2-naphthochinon-(1)-diazid-4-sulfonsäure-(K-Salz) und 1 Mol Kristallwasser.

Das reine 7-Hydroxy-1,2-naphthochinon-4-sulfonsäure-(K-Salz) erhielt man durch Umlösen des Rohprodukts aus Wasser.
- Ausbeute:: 148 g Rohprodukt (89 %ige Qualität) =131,7 g Reinprodukt (100 %ige Qualität), d.h. 82,4 % d. Theorie

### Kenndaten:

- Aspekt:: dunkelrotes kristallines Pulver
- MP:: ≧ 275 °C (Z)

### Elementaranalyse

| Bruttoformel: C₁₀H₅O₆SK x 1H₂O | | | | | |
|---|---|---|---|---|---|
| MG: | 310 | | | | |

| | C | H | S | H₂O | |
|---|---|---|---|---|---|
| Ber.: | 38,7 | 2,3 | 10,3 | 5,8 | (%) |
| Gef.: | 38,8 | 2,1 | 9,9 | 6,1 | (%) |

### 7-Hydroxy-1,2-naphthochinon-(2)-diazid-4-sulfonsäure (K-Salz) x 1 H₂O (IV)

131,4g (0,377 Mol) 89 %iges 7-Hydroxy-1,2-naphthochinon-4-sulfonsäure (K-Salz) x 1 H₂O wurden unter Rühren in eine Suspension aus 88,2 g (0,47 Mol) p-Toluolsulfonsäurehydrazid und 900 ml Methanol innerhalb von 25 Minuten portionsweise eingetragen und 3 Stunden bei 20 - 28 °C weitergerührt, wobei sich die dunkelrote Suspension in eine gelbe umwandelte. Diese wurde anschließend auf 0 bis 5 °C abgekühlt, das rohe gelbe Reaktionsprodukt abgesaugt, auf der Filternutsche zweimal mit je 100 ml Ethanol gewaschen und der Filterrückstand 16 Stunden im Umlufttrockenschrank bei 50 - 55 °C getrocknet. Das rohe Reaktionsprodukt enthielt geringe Anteile an 7-Hydroxy-1,2-naphthochinon-(1)-diazid-4-sulfonsäure (K-Salz) und 1 Mol Kristallwasser. Durch Umlösen des Rohprodukts aus Ethanol/Wasser wurde das reine 7-Hydroxy-1,2-naphthochinon-(2)-diazid-4-sulfonsäure (K-Salz) erhalten.
- Ausbeute:: 112,5 g Rohprodukt (95,2 %ige Qualität) =107,1 g Reinprodukt (100 %ige Qualität), d.h. 93,5 % d. Theorie

### Kenndaten:

- Aspekt:: hellgelbes kristallines Pulver
- MP:: ≧ 165 °C (Z)

### Elementaranalyse

| Bruttoformel: C₁₀H₅N₂O₅SK x 1H₂O | | | | | |
|---|---|---|---|---|---|
| MG: | 304 | | | | |

| | C | H | N | S | H₂O |
|---|---|---|---|---|---|
| Ber.: | 37,3 | 2,2 | 8,7 | 9,9 | 5,6 |
| Gef.: | 37,0 | 2,0 | 8,5 | 9,6 | 5,3 |

In der folgenden Tabelle 1 wurden einige der erfindungsgemäßen Verbindungen der allgemeinen Formel Z zusammengestellt.

| Lfd. Nr. | X | Fp (°C) | Elementar-Analyse | | | | | |
|---|---|---|---|---|---|---|---|---|
| | | | | C | H | N | S | H₂O |
| 1 | K | ≧ 165 (Z.) | B. | 37,3 | 2,2 | 8,7 | 9,9 | 5,6 |
| | | | G. | 37,0 | 2,0 | 8,5 | 9,6 | 6,3 |
| 2 | NH₄ | ≧ 170 (Z.) | B. | 42,4 | 3,2 | 14,8 | 11,3 | - |
| | | | G. | 41,3 | 3,1 | 14,8 | 11,2 | - |

## Patentansprüche

1. Verfahren zur Herstellung von Verbindungen der allgemeinen Formel Z 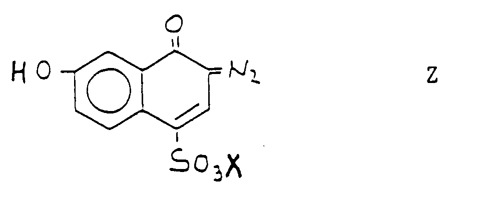 in der
X = Wasserstoff, Metall oder eine Ammoniumgruppe bedeutet, dadurch gekennzeichnet, daß man
1) 2,7-Dihydroxynaphthalin nitrosiert,
2) das entstandene 2,7-Dihydroxy-1-nitrosonaphthalin (I) mit Alkalihydrogensulfit sulfoniert und die gebildete Bisulfit Additionsverbindung in saurer Lösung zur 2,7-Dihydroxy-1-aminonaphthalin-4-sulfonsäure (II) reduziert,
3) diese zur 7-Hydroxy-1,2-naphthochinon-4-sulfonsäure (III) oxidiert und als Salz abscheidet und
4) dieses Salz mit einem Arylsulfonsäurehydrazid zu dem entsprechenden Salz der 7-Hydroxy-1,2-naphthochinon-(2)-diazid-4-sulfonsäure (IV) umsetzt und dieses isoliert.

2. Verfahren nach Anspruch 1, dadurch gekennzeichnet, daß man die Nitrosierung in essigsaurer wäßriger Suspension mit Alkalinitrit bei Temperaturen zwischen + 5 und - 10 °C durchführt und die Nitrosoverbindung isoliert.

3. Verfahren nach Anspruch 1, dadurch gekennzeichnet, daß man die Sulfonierung mit Alkalihydrogensulfit oder Alkalidisulfit in wäßriger Phase bei einem pH-Bereich von 5 - 7 durchführt und die entstandene Bisulfit-Additionsverbindung ohne Zwischenisolierung in mineralsaurer wäßriger Lösung bei Temperaturen zwischen 20 und 60 °C zu der entsprechenden Aminosulfonsäure reduziert.

4. Verfahren nach Anspruch 1, dadurch gekennzeichnet, daß man die Oxidation mit wäßriger Salpetersäure von 15 - 25 Gewichtsprozent bei Temperaturen zwischen 15 und 25 °C durchführt und die entstandene Naphthochinonsulfonsäure als Salz, vorzugsweise als Kalium- oder als Ammoniumsalz, abscheidet und isoliert.

5. Verfahren nach Anspruch 1, dadurch gekennzeichnet, daß man das Salz der Naphthochinonsulfonsäure mit p-Toluolsulfonsäurehydrazid in organischer Phase, vorzugsweise in Methanol, bei Temperaturen zwischen 20 und 70 °C, zu dem entsprechenden Salz der 7-Hydroxy-1,2-naphthochinon-(2)-diazid-4-sulfonsäure umsetzt und dieses isoliert.

6. Verfahren nach Anspruch 1, dadurch gekennzeichnet, daß X in der allgemeinen Formel Z Alkali- oder Erdalkalimetall bedeutet.

7. Verfahren nach Anspruch 1 oder 6, dadurch gekennzeichnet, daß X in der allgemeinen Formel Z Natrium, Kalium oder die Ammoniumgruppe bedeutet.

8. Verwendung der nach Ansprüchen 1 bis 7 hergestellten Verbindungen als Zwischenprodukte für die Herstellung lichtempfindlicher Verbindungen in strahlungsempfindlichen Gemischen und Materialien.

## Claims

1. A process for preparing a compound of the general formula Z 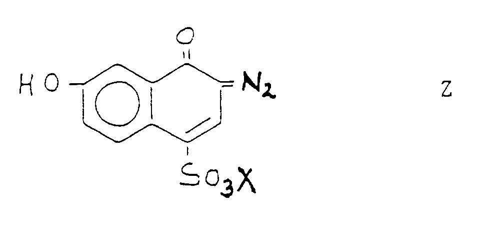 in which
X denotes hydrogen, a metal or an ammonium group, comprising the steps of
1) nitrosating 2,7-dihydroxynaphthalene,
2) sulfonating the resulting 2,7-dihydroxy-1-nitrosonaphthalene (I) with an alkali metal hydrogen sulfite and reducing the bisulfite addition compound formed in acidic solution to 2,7-dihydroxy-1-aminonaphthalene-4-sulfonic acid (II),
3) oxidizing the latter to 7-hydroxy-1,2-naphthoquinone-4-sulfonic acid (III) and precipitating it as a salt and
4) converting this salt with an arylsulfonic acid hydrazide to the corresponding salt of 7-hydroxy-1,2-naphthoquinone-2-diazide-4-sulfonic acid (IV) and isolating this salt.

2. The process as claimed in claim 1, wherein the nitrosation is carried out in an agueous suspension containing acetic acid with an alkali metal nitrite at temperatures between + 5, and - 10°C and the nitroso compound is isolated.

3. The process as claimed in claim 1, wherein the sulfonation is carried out with an alkali metal hydrogen sulfite or alkali metal disulfite in agueous phase in a pH range of 5 - 7 and the bisulfite addition compound formed is reduced without intermediate isolation in an aqueous solution containing a mineral acid at temperatures between 20 and 60°C to the corresponding aminosulfonic acid.

4. The process as claimed in claim 1, wherein the oxidation is carried out with 15 - 25 percent by weight aqueous nitric acid at temperatures between 15 and 25°C and the naphthoquinone-sulfonic acid formed is precipitated and isolated as a salt, preferably as the potassium or ammonium salt.

5. The process as claimed in claim 1, wherein the salt of the naphthoquinone-sulfonic acid is converted with p-toluenesulfonic acid hydrazide in an organic phase, preferably in methanol, at temperatures between 20 and 70°C to the corresponding salt of 7-hydroxy-1,2-naphthoquinone-2-diazide-4-sulfonic acid and this salt is isolated.

6. The process as claimed in claim 1, wherein X in the general formula Z is an alkali metal or alkaline earth metal.

7. The process as claimed in claim 1 or 6, wherein X in the general formula Z is sodium, potassium or the ammonium group.

8. The use of a compound prepared as claimed in any of claims 1 to 7 as an intermediate for the production of light-sensitive compounds in radiation-sensitive mixtures and materials.

## Revendications

1. Procédé pour la préparation de composés de formule générale Z 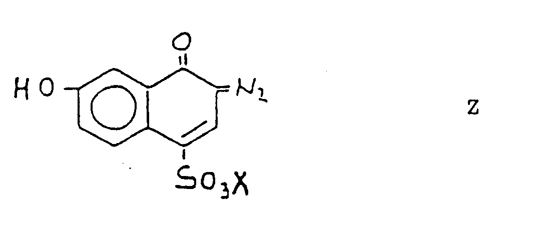 dans laquelle
X représente un atome d'hydrogène ou un métal, ou le groupe ammonium,
dans lequel
1) on soumet à une nitrosation du 2,7-dihydroxynaphtalène,
2) on soumet à une sulfonation avec un hydrogénosulfite alcalin le 2,7-dihydroxy-1-nitrosonaphtalène (I) formé et on réduit le composé d'addition bisulfite formé, en solution acide, pour aboutir à l'acide 2,7-dihydroxy-1-aminonaphtalène-4-sulfonique (II),
3) on oxyde ce dernier en l'acide 7-hydroxy-1,2-naphtoquinone-4-sulfonique (III) et on le sépare sous forme de sel, et
4) on fait réagir ce sel avec un arylsulfonhydrazide, pour aboutir au sel correspondant de l'acide 7-hydroxy-1,2-naphtoquinone-(2)-diazide-4-sulfonique (IV), et on isole celui-ci.

2. Procédé selon la revendication 1, caractérisé en ce que l'on effectue la nitrosation avec un nitrite alcalin, dans une suspension aqueuse acidifiée par de l'acide acétique, à des températures comprises entre +5 et -10°C, et on isole le composé nitroso.

3. Procédé selon la revendication 1, caractérisé en ce que l'on effectue la sulfonation avec un hydrogénosulfite alcalin ou un disulfite alcalin en phase aqueuse, dans un intervalle de pH de 5 à 7, et on réduit sans isolement intermédiaire le composé d'addition bisulfite obtenu en l'acide aminosulfonique correspondant, dans une solution aqueuse acidifié par un acide minéral, à des températures comprises entre 20 et 60°C.

4. Procédé selon la revendication 1, caractérisé en ce que l'on effectue l'oxydation avec une solution aqueuse d'acide nitrique à 15-25 % en poids, à des températures comprises entre 15 et 25°C, et l'acide naphtoquinonesulfonique formé est séparé sous forme de sel, de préférence sous forme de sel d'ammonium ou de potassium, et isolé.

5. Procédé selon la revendication 1, caractérisé en ce que l'on fait réagir le sel de l'acide naphtoquinonesulfonique avec du p-toluènesulfonhydrazide en phase organique, de préférence dans du méthanol, à des températures comprises entre 20 et 70°C, pour aboutir au sel correspondant de l'acide 7-hydroxy-1,2-naphtoquinone-(2)-diazide-4-sulfonique, et on isole celui-ci.

6. Procédé selon la revendication 1, caractérisé en ce que X dans la formule générale Z représente un métal alcalin ou alcalino-terreux.

7. Procédé selon la revendication 1 ou 6, caractérisé en ce que X dans la formule générale Z représente le sodium, le potassium ou le groupe ammonium.

8. Utilisation des composés préparés selon les revendications 1 à 7, en tant que produits intermédiaires pour la préparation de composés photosensibles dans des compositions et matériaux sensibles aux radiations.
